(19) Europäisches Patentamt
European Patent Office
Office européen des brevets

(11) **EP 2 685 424 A2**

(12) **EUROPEAN PATENT APPLICATION**

(43) Date of publication:
**15.01.2014 Bulletin 2014/03**

(51) Int Cl.:
***G06T 7/00*** *(2006.01)*

(21) Application number: **13174900.4**

(22) Date of filing: **03.07.2013**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**

(30) Priority: **10.07.2012 EA 201200924**

(71) Applicant: **Zakrytoe Akcionernoe Obshchestvo
"Impul's"
St.Petersburg 197343 (RU)**

(72) Inventor: **Afanasenko, Arseniy Sergeevich
199155 Saint-Petersburg (RU)**

(74) Representative: **Anohins, Vladimirs et al
Agency Tria Robit
P.O. Box 22
1010 Riga (LV)**

(54) **Method for acquisition of subtraction angiograms**

(57)     The invention relates to the field of digital processing of X-ray images and can be used in digital subtraction angiography to compensate for impact of involuntary patient movement and movement of internal organs on image of the vascular system.

The technical result of the claimed invention is the improvement of diagnostic value of subtraction angiographic images by eliminating artifacts caused by the motility of anatomical structures.

Technical result is achieved by the that at the stage of digital images registration a search for characteristic details is performed for each image. According to the shift of the said details determine expectable shift of a patient organs. Then perform segmentation of image from the pre-contrast series in the region of homogenous warping ; for each region a geometrical transformation is calculated and corresponding geometrical transformations are performed for each region of a series of pre-contrast digital images.

Fig. 4

**Description**

*Field of the invention*

The invention relates to the field of digital X-ray image processing and can be applied to digital subtraction angiography aimed at compensating the impact of patient's involuntary movement and its internal organs normal movement on images of the vascular system.

*Previous state of art*

An angiography is a medical imaging technique used to visualize the inside of blood vessels based on injecting a contrast medium into the patient's blood vessels under examination. Besides vessels in the angiograms can be seen the surrounding organ and tissue images that often interfere with recognizing vessels filled with contrast medium. In order to make surrounding tissue less visible in digital subtraction angiography (DSA) they generate a pre-contrast image that is an X-ray image of a patient's region of interest (ROI) obtained before contrast agent to be injected into vessel system. Then, a sequence of images is taken to show the passage of injected contrast agent through vessels of interest. These images being called post-contrast images are those images that are registered at the beginning of contrast medium injection; they represent phases of blood vessels contrast medium fulfillment, along blood vessels advancement and gradual in blood dissolution. Due to subtraction of the post-contrast image from the pre-contrast image the  artifacts not belonging to vessel system could be corrected. This means vessels visibility enhancement and treatment and diagnostic improvement.

Since acquisition of post-contrast and pre-contrast images is performed at different time points any space change of surrounding tissue and organs cause artifacts in images. They are especially visible on the contrast borders of the movable organs. In some cases the contrast of artifacts exceeds that of blood vessels which leads to masking essential fragments of the vessel system and makes some details unrecognizable.

It is known a method for matching images (US№7409078) applied in DSA. According to this method a DSA image is generated as follows:

- obtain pre-contrast image before contrast agent injection into patient's vessel system;
- inject contrast agent into patient's vessel system;
- obtain post-contrast image;
- place a regular grid on pre-contrast image;
- match post-contrast and pre-contrast images using control points in the grid crossings;
- calculate a subtracted image which is to be displayed on the monitor;
- test control points stability; if it is not sufficient divide the grid into smaller or bigger cells.

The method suffers from incomplete set of processed image characteristics; this leads to suboptimal correction of motion artifact.

It is known a method and device for DSA images (USNo5848121). According to this method a DSA image is generated as follows:

- obtain pre-contrast image before contrast agent injection into patient's vessel system;
- inject contrast agent into patient's vessel system;
- obtain post-contrast image;
- find a set of particular details in pre-contrast and post-contrast images;
- determine correspondence between identified particular details in both images;
- build a model of adaptive local geometrical transform based on characteristic point coordinates ;
- apply obtained transform to deform pre-contrast image;
- build a subtracted image by means of subtracting the logarithm of deformed pre-contrast image from the logarithm of post-contrast image.

The method's shortcoming is that fallacious determination of characteristic point correspondence of pre-contrast and post-contrast images leads to irremovable defects in the resulted image.

The closest method to the disclosed herein is the method of matching, subtraction and displaying chest X-ray images according to the patent application USNo2010/0266188. The method consists in the following operations:

- obtain pre-contrast image before contrast agent injection into patient's vessel system;
- inject contrast agent into patient's vessel system;

- obtain post-contrast image;
  thus, at least two X-ray images visualizing the same organs are obtained;
- perform preliminary image processing involving normalization according to the size, intensity and color depth, as well as ROI selection;
- perform rough image matching including shift and rotation compensation;
- perform precise image matching, using correlated matching of small details or methods for optical flow computation;
- subtract matched images in a way to provide the best visibility of required details and the best removal of the extraneous details.

Common with the claimed method features are:

1. Estimation of geometrical transforms connecting matched images over limited set of control regions.
2. Possibility to compensate shift, rotation and local warping of examined organs in the image.
3. Multiscale analysis of images at motion estimation.
4. Subtraction processing of matched images aimed at special detail enhancement (vessels in the claimed invention or lung micronodules in the prototype).

The main disadvantage of the method claimed in US №2010/0266188 is limited accuracy of motion estimation when the examined object has specific characteristics which considerably effects quality of obtained angiogram. This is because of sequential process of motion compensation at different scales, from large scale (rough) towards smaller scale (more accurate). As spatial frequency analysis of multiplicity of images encountered in clinical practice shows many images contain essential details that result in reduction of spatial resolution only. In these cases the said method provides accurate results till a definite moment after which it results in error accumulation.

Besides, according to the known method the function of coordinate transformation is smooth within the ROI what adversely affects the motion compensation precision provided at least two moving fragments with clearly defined edges are in the ROI.

## Disclosure of the invention

The technical result of the present invention consists in increase of diagnostic value of subtraction angiogram due to removal of moving anatomical structures artifacts.

The technical result of the method for acquisition of subtraction angiogram CONSISTING IN performing by means of X-ray device an X-ray exposure of a patient resulting in acquisition of a series of N pre-contrast digital images, injecting contrast agent into patient's vessel system and obtaining after an exposure post-contrast image series of M digital images; further matching spatial correspondence of digital pre-contrast and post-contrast image series; subtracting pre-contrast images from post-contrast ones; and transferring obtained digital angiogram to output device is ACHIEVED by that at the stage of image matching the search of characteristic details in each image is performed; referring to the shifts of the said details likely shifts of patient's organs are determined; image segmentation of pre-contrast image series within the similar motion area is performed; geometrical transformation for each area is calculated; appropriate geometrical transformation for all areas in pre-contrast image series is applied.

Another possible embodiment of the present invention wherein as characteristic details such elements of bone structures and patient's organs are selected in a way that their position in digital images during an exposure is definitely determined regardless of change in their position in space.

Another possible embodiment of the present invention wherein at segmentation of pre-contrast image series within the similar motion area a finite set of possible shifts against the current image of post-contrast series is ascribed to each characteristic detail further, areas within which characteristic details move in concord are selected.

Another possible embodiment of the present invention wherein in pre-contrast image series unmovable parts of X-ray equipment are selected; and at the stage of digital image matching these areas are given a zero-shift.

Another possible embodiment of the present invention wherein after acquisition of pre-contrast series of N digital images a single referring pre-contrast image by means of weighted summation of digital images is generated, wherein the first one is stored without changes and, for each subsequent image before summation with current reference image spatial matching with the latter is performed.

Another possible embodiment of the present invention wherein during acquisition of a series of post-contrast digital images the reference image from post-contrast series is selected; wherein this image is subjected to characteristic detail search, shift estimation and segmentation repeatedly.

Another possible embodiment of the present invention wherein in each of obtained digital images organs and tissues shadowing each other are divided into at least two overlapped images which are the slices corresponding to organs and tissues of different depth; image matching is performed independently for each slice with subsequent summation.

## *Detailed description of the invention*

The claimed solution, as well as the potential of its practical implementation and accomplishment of its practical objective, outlined above are illustrated in figures 1 through 4.

Fig. 1 shows X-ray device;
Fig. 2 shows the content of post-contrast image series;
Fig.3. shows segmentation of moving organs;
Fig. 4. shows processing results.

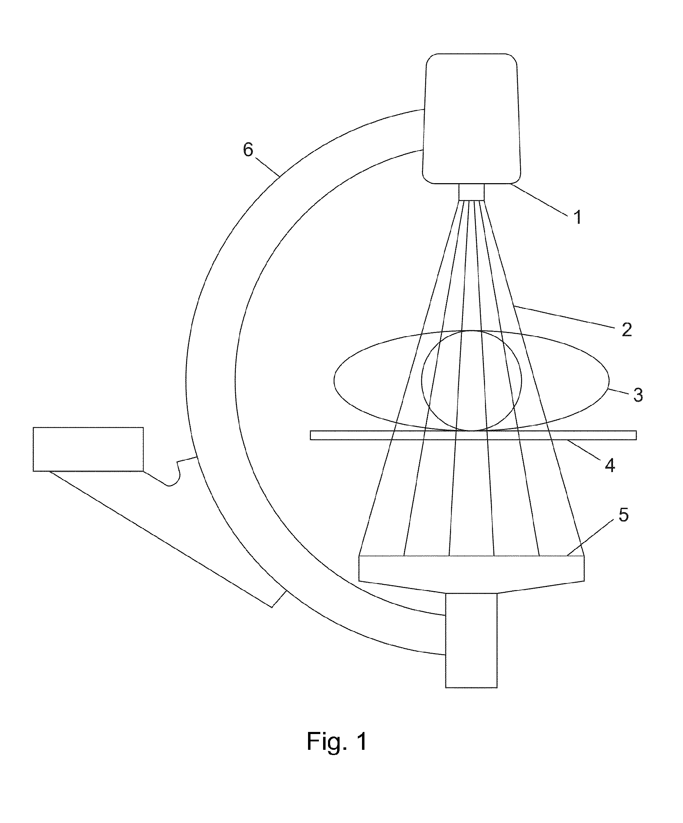

Acquisition of digital X-ray images is performed, for example, by means of the X-ray device shown in Fig. 1. It comprises X-ray tube 1 which emits a flow of X-rays 2. X-rays 2 go through the patient's body 3 placed on the table 4 and enter the receiver 5. The receiver 5 provides conversion of X-rays into a digital image. In one of the possible versions of embodiment the receiver can comprise a scintillating screen (not shown) converting X-rays into visible light and a photosensitive matrix array (not shown). The X-ray tube 1 and the receiver 5 are fixed on the support 6 having 4 degrees of freedom concerning motion against the table.

According to the claimed method patient's exposure resulted in acquisition of pre-contrast series of N digital images (where N ≥ 1). Having injected contrast agent into patient's vessel system one can obtain post-contrast series of M digital images (where M ≥ 1). For every image of post-contrast series one can select an appropriate reference image which can belong to either pre-contrast or post-contrast series or be a result of combined processing of a number of previous images from both the series.

According to different types of the invention embodiments the following versions of acquisition and processing images of pre-contrast and post-contrast series.

1. A pre-contrast series of N digital images (where N ≥ 1) is obtained while support is motionless. The image of the highest quality selected manually (or a single pre-contrast image, N = 1) is used as a reference image during processing of all images from post-contrast series.

2. A pre-contrast series of at least two digital images is obtained while support is motionless. The image of the highest quality selected manually is saved as a reference image. All subsequent images undergo spatial matching with the reference image. Matched images are subject to summation, the result of which is saved as an updated reference image. This results in reference image denoising. Only one reference image is used when processing post-contrast image series.

3. In case angiographic examination requires to move support for example, when examining patient's lengthy vessels, pre-contrast image are obtained at different support positions. Support motion path are identical for both pre-contrast and post-contrast image series. Each pre-contrast image is a referring one at processing post-contrast image series obtained in the same support position.

4. In cases of post-contrast image series acquisition an unforeseen support or patient motion happened or other reasons have changed the image content, from now as a preference image will be instead of pre-contrast image one of the post-contrast images processed in a way to lower visibility of the vessels filled with contrast agent.

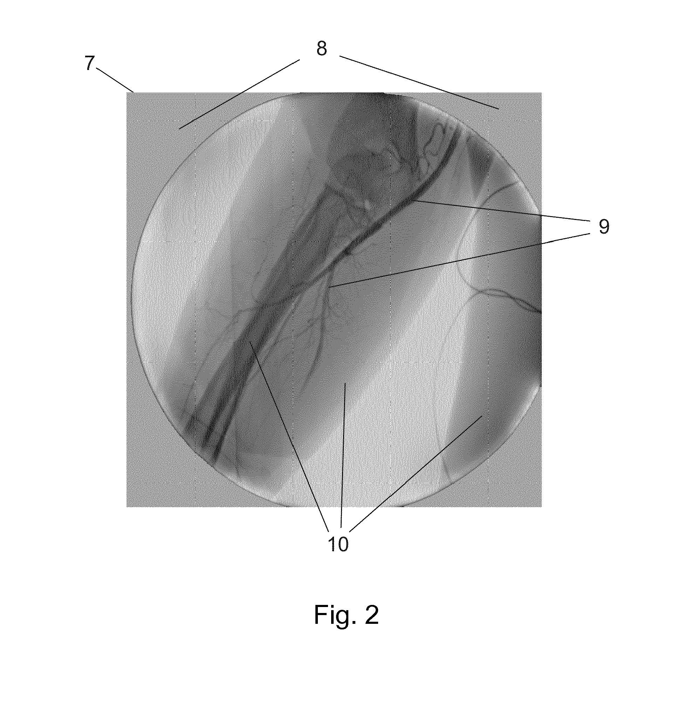

Fig. 2 shows the content of digital angiographic image of post-contrast series. The rectangular field of view 7 of the receiver is in part concealed by X-ray device components 8 motionless during examination. Patient's image includes filled with contrast agent vessels 9 as well as organs and tissues 10 not being involved in angiographic examination.

To match post-contrast series and reference image one can determine a geometric transformation providing the best way of the images matching. Due to preliminary selection of motionless regions a sector to match images is narrowed. It is required when a part of the receiver's field of view is shielded by collimator shutters or the receiver includes an electro-optical transducer of circular section. The edges of motionless regions have a shape of linear or smooth curves as well as high contrast; this makes it possible to use for their search a well-known algorithms of geometric primitives selection (e.g., Hough transformation) in combination with the regression analysis technique.

Immobility of this regions makes it possible to select them once during processing of each reference image.

When performing image registration firstly select finite set of control regions from the reference image. Control regions are selected in such a way as to contain characteristic details of the image. Characteristic detail shall have such properties that make it possible to reliably discern it in the images of the given object regardless of brightness and geometric transformations as well as in the presence of noise.

To select control regions the following operations are performed:

1. Multiscale image representation by means of Gaussian pyramid or similar transformation. The number of scales is set manually or coordinated with the spatial-frequency spectrum shape of the image.

2. At each scale a characteristic details enhancement operator is applied to the image. In one embodiment of the invention this operator is based on the calculation of the covariance matrix on texture at each point of the image. Detector response is the product of the eigenvalues of this matrix. Local maxima of the detector response correspond to the brightness difference, bending and intersections of objects' boundaries.

3. Limit the set of characteristic details by selecting details with the maximum detector response value, found at different scales. To correctly compare the detector response values at different scales a monotone increasing correction factor which increases when performing transition from a larger to a smaller scale is introduced.

4. In the neighborhood of each of the characteristic details found form the control region, the size of which corresponds to the scale at which the maximum detector response obtained. To each region can be further attributed a spatial orientation corresponding to the resultant eigenvector of the texture covariance matrix.

A simplified embodiment of the invention is possible, when the search for characteristic details is not performed. Instead, a geometrical grid of preset shape (e.g. squared) is superimposed on the image and the set of control regions is located in the grid nodes.

The result of the reference image analysis is a set of control regions, for each of which the coordinates of the center, the size and, depending on the embodiment of the invention, the additional attributes are saved.

For each digital image from the post-contrast set, as well as for the images from pre-contrast set which are non-reference, the procedure of registration with the reference image is performed, consisting of the following:

1. Select control regions in the image in the same way as when processing the reference image.

2. Perform a comparison of control region attributes of the current image with the saved control region attributes of the reference image. Based on the correlation determine a one-to-one correspondence between control regions. As a result to each control region a displacement vector is attributed relative to the reference image. In the general case, the set of control regions selected in the reference image may not fully match with the set of control regions of the current image. Shifting determination errors which arise during the above mentioned operations are eliminated by applying statistical technique during further processing.

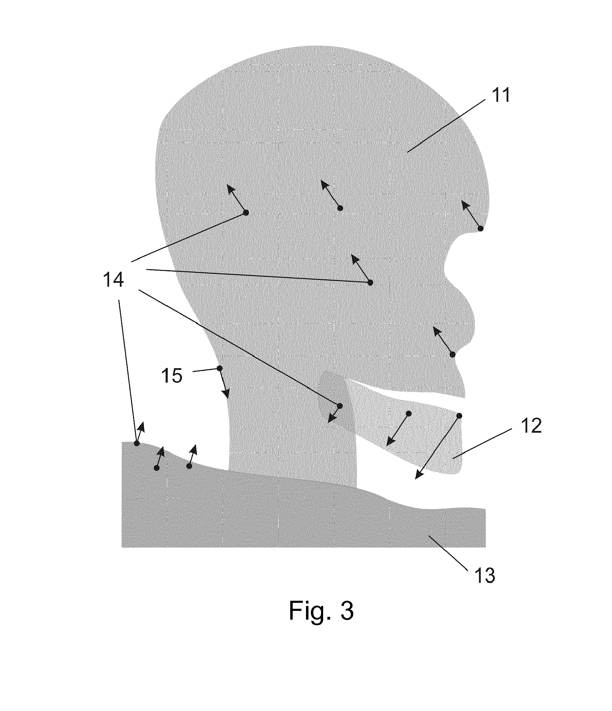

3. From the set of control regions select such groups, shift of which occurs consistently and is described with satisfactory accuracy by the model chosen for the geometric transformation. In one possible embodiment of the invention, such a model is an affine transformation, which implies various combinations of shift, rotation, tilting and zooming. For example, FIG. 3 shows the segmentation (partitioning) of the lateral projection of the patient's head into three areas with different motility: the skull 11, the lower jaw 12 and the shoulders 13. The displacement vectors are attributed to the centers of control region 14, including outlier vectors 15. As a result of the cluster analysis of shifts to each group of control regions is attributed a general model of geometric transformations.

4. Determine the boundaries of image segments containing selected groups of control regions, using data on image brightness gradient.

5. Transforming the reference image, applying to the selected image segments calculated geometric transformations. At the boundaries of the segments interpolation can be applied to reduce edge effects.

After image registration is finished a pixel-wise arithmetical division of the current image brightness by the brightness of the transformed reference image is performed. This operation corresponds to the logarithmic subtraction. The result is the required subtraction image.

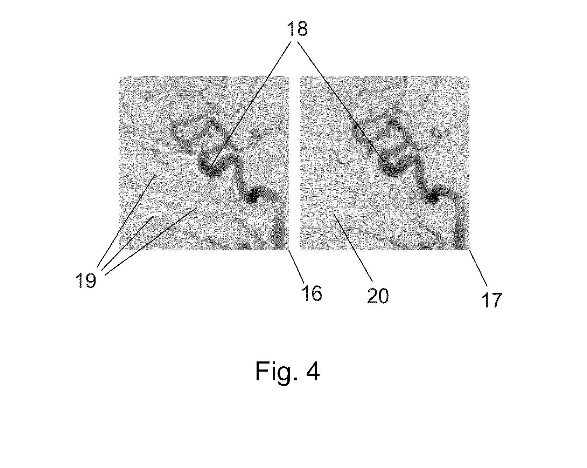

FIG. 4 shows a comparison of angiographic image 16, obtained without the use of motion compensation, and the angiographic image obtained by the claimed method 17. In the first case, the images of vessels 18 filled with contrast agent contain motion artifacts 19 not being an images of any existing organs or tissues. In the second case the artifacts 20 expressed significantly weaker.

Due to the segmentation of the optic flow field obtained after comparison of the control regions of the reference image and the current image, achieve lower residual visibility of organs which worsens discernibility of vessels studied, and improves the diagnostic value of the angiographic images, obtained by the claimed method.

**The best embodiment of the invention**

X-ray images are obtained through the use of X-ray system, fixed parts of which are not projected onto the working field of detector. X-ray exposure is performed to obtain a series of 2-3 pre-contrast digital images. In the first digital image from the series of pre-contrast images a search of characteristic details is performed. To do this, the following operations are performed:

1. Multiscale representation of the image is formed, consisting of 4 levels. The first level is the image itself. Then in order to form each next level a digital smoothing filter having the aperture of $3 \times 3$ elements is applied, and the image is subsampled by the factor of 2.

2. At each level of the multiscale image select characteristic details. To do this, first calculate a first partial derivatives of brightness horizontally and vertically:

$$I'_x = \frac{\partial I}{\partial x}, \ I'_y = \frac{\partial I}{\partial y}. \tag{1}$$

Then for each pixel of the image covariance matrix of the texture is computed:

$$\mathbf{C} = \begin{bmatrix} g * \left( I'_x I'_x \right) & g * \left( I'_x I'_y \right) \\ g * \left( I'_x I'_y \right) & g * \left( I'_y I'_y \right) \end{bmatrix}, \tag{2}$$

where g - the impulse response of Gaussian smoothing filter with an aperture of $5 \times 5$ elements. Then edge enhancement operator response is calculated, scale-weighted:

$$h = \sqrt{2^n} \left( \det \mathbf{C} - 0,02 \, \mathrm{tr} \, \mathbf{C} \right), \tag{3}$$

where $n \in [0,3]$ - the number of the current level of the multiscale representation, det $\mathbf{C}$ - the determinant of the covariance matrix of the texture, tr $\mathbf{C}$ - trace of the covariance matrix of the texture. Then select such points in the image for which the value of $h$ is a local maximum within the neighborhood of radius $r$. The value of $r$ is assigned to be equal to 2% of the image minimum linear size (rounded to the nearest whole number).

3. Combine the results of characteristic details selection at all levels of the multiscale representation and form the initial set of characteristic details. As this takes place the coordinates and value of the local maximum of h, as well as the number the multiscale representation level (at which current detail was selected) are attributed to each characteristic detail.

Exclude from the set those details close to which on other levels there are details with a higher value of $h$ (radius of the neighborhood to eliminate recurring details is set to 1% of the image minimum linear size). The resulting set of values are sorted in descending order of value h and keep in the final set no more than the first 100 details. Every detail is assigned the control region, the size of which is smaller, the larger the scale at which the characteristic detail is selected.

Radiographic contrast medium is injected into the patient's blood vessels under examination and X-ray exposure is performed which results in a series of M post-contrast digital images (where M ≥ 1)

For a group of images, including at least one image from the pre-contrast series and all images from the post-contrast series, perform image registration, consisting of the following operations:

1. Multiscale representation of the image is formed, consisting of 4 levels. The first level is the image itself. Then in order to form each next level a digital smoothing filter having the aperture of $3 \times 3$ elements is applied, and the image is subsampled by the factor of 2.

2. For each control region the shift and rotation are determined by means of correlation method. The search begins at that level of multiscale representation, which corresponds to the size of the characteristic detail, and continue at a larger scale, successively reducing the search range and increasing accuracy. The search consists in the maximization of the cross-correlation coefficient between the patches of the reference and the current image:

$$\left[ \begin{array}{ccc} \hat{dx}, & \hat{dy}, & \hat{\alpha} \end{array} \right] = \arg\max \frac{\text{cov}\left\{ T^R, \text{warp}1\left( I^R, dx, dy, \alpha \right) \right\}}{\sqrt{D\left\{ T^R \right\} D\left\{ \text{warp}1\left( I^R, dx, dy, \alpha \right) \right\}}}, \qquad (4)$$

where $T^R$ - patch of the reference image $R$, corresponding to the control region, $I^R$ - patch of the current image $I$, corresponding to the control region, warp1($I, dx, dy, \alpha$) - transformation of the image $I$, consisting of the translation by the vector ($dx, dy$) and rotation by the angle $\alpha$, cov - covariance of brightness samples of current and reference images within the patch , $D$ - dispersion.

3. Perform segmentation of the control regions into K segments in such a way that for each segment the shifts of control regions are described by an affine transformation with the least error. Affine transformation of coordinates has the following form:

$$\left[ \begin{array}{cc} x' & y' \end{array} \right] = \left[ \begin{array}{ccc} x & y & 1 \end{array} \right] \mathbf{A}, \quad \mathbf{A} = \begin{bmatrix} a_1 & a_4 \\ a_2 & a_5 \\ a_3 & a_6 \end{bmatrix}, \qquad (5)$$

where $x, y$ - reference coordinates of the point, $x', y'$ - *converted coordinates,* $a^1 ... a^6$ - conversion factors. Error for each segment is determined as:

$$\varepsilon_k = \sum_{i \in P_k} \left\| \left[ \begin{array}{cc} x_i + dx_i & y_i + dy_i \end{array} \right] - \left[ \begin{array}{ccc} x_i & y_i & 1 \end{array} \right] \mathbf{A}_k \right\|, \qquad (6)$$

where $k \in [1, K]$ - segment number, $i$ - control region number, $P_k$ - a subset of control regions, relating to $k$-th segment. The goal of segmentation is to minimize the total error:

$$\sum_{k=1}^{K} \varepsilon_k \to \min. \qquad (7)$$

In the process of minimization of the formula (7) a $P_k$, disjoint subsets are determined which make up an exhaustive set of control regions; also the corresponding affine matrix $\mathbf{A}_k$ is determined.

The number of $K$ segments is initially unknown, but to improve segmentation procedure convergence this number is limited to the value of 5. During the iterative process of optimization the segments with similar transformation matrices are combined, thereby the final number of segments, depending on the nature of the control regions motility ranges from 1 to 5.

Perform the processing of the current image taking into account the motion. If the current picture is from a pre-contrast series, it is subjected to inverse transformation, calculated at the previous step. The reference image is updated by a weighted arithmetic summation with the modified current image:

$$R' = R + \frac{1}{t} \left( \text{warp}2\left( I_t, \mathbf{A}_1, \mathbf{A}_2, ..., \mathbf{A}_K \right) - R \right), \quad t > 0 \qquad (8)$$

where $R'$ - reference image after the update , $R$ - reference image before the update, $t$ - image number in the processed sequence, $I_t$ - current image, warp2($I_t, \mathbf{A}_1, \mathbf{A}_2, ..., \mathbf{A}_K$) - joint transformation, which results in that each segment of the image $I_t$ is subjected to affine transformation, defined by the $\mathbf{A}_K$ matrix.

If the current picture is from the post-contrast series it is subjected to subtraction. The reference is subjected

EP 2 685 424 A2

to transformation, calculated at the previous step. Weighted logarithm of the modified reference image is subtracted from the logarithm of the current image:

$$F = \ln I - s \ln \left( \mathrm{warp}\,2 \left( R, \mathbf{A}_1, \mathbf{A}_2, \ldots \mathbf{A}_K \right) \right), \qquad (9)$$

where s - subtraction factor within the range from 0 to 1. The obtained image *F* is displayed on the output device. By adjusting *s*, radiologist can make visible on the output device not only studied vessels but surrounding organs which serve as an anatomical landmarks.

The diagnostic value of obtained angiographic image is improved by removing artifacts caused by the motility of anatomical structures.

**Claims**

1. A method for obtaining angiographic images, which consists in that with the use of X-ray system exposure of a patient is performed which results in the series of N pre-contrast digital images, then a contrast agent is injected into the blood vessels of patient under examination, then an X-ray exposure is performed which results in the series of M post-contrast digital images whereupon a spatial image registration of digital images from pre-contrast and post-contrast series is performed, then digital images from pre-contrast series are subtracted from the digital images from post-contrast series and the resultant digital angiographic image is transmitted to the output device, **CHARACTERIZED IN THAT** at the stage of digital images registration a search for characteristic details is performed for each image, according to the shift of the said details determine expectable shift of a patient organs, then perform segmentation of image from the pre-contrast series in the region of homogenous warping; for each region a geometrical transformation is calculated and corresponding geometrical transformations are performed for each region of a series of pre-contrast digital images.

2. The method of claim 1, wherein, in the capacity of the characteristic details select such bone structure parts and organs of a patient, position of which in the digital images can be uniquely determined regardless of their spatial position.

3. The method of claim 1, **characterized in that** when segmenting a digital image from pre-contrast series in the region of homogenous warping, a finite set of possible shifts relative to current image from post-contrast series is attributed to each characteristic detail and select regions in the image where characteristic details shift concordantly.

4. The method of claim 1, **characterized in that** in the images from pre-contrast series select fixed parts of X-ray equipment and at the stage of digital images registration a zero bias is attributed to these regions.

5. The method of claims 1 - 4, **characterized in that** after obtaining a series of N pre-contrast digital images form a singular reference pre-contrast digital image by weighted summation of digital images first of which is saved without modifications and for each subsequent image before summation with the current reference image perform spatial registration with it.

6. The method of claims 1 - 4, **characterized in that** in the process of obtaining a series of post-contrast digital images select a reference image from the series of post-contrast images also and repeat the search for characteristic details in it, assess shift and segmentation.

7. The method of claims 1 - 4, **characterized in that** in each of the obtained digital images organs and tissues which shade each other, separated by at least two overlapping images - layers corresponding to organs and tissues located at different depths, images registration is performed independently for each layer, and the results are then summed.

Fig. 1

Fig. 2

Fig. 3

Fig. 4

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20100266188 A